# EUROPÄISCHE PATENTANMELDUNG

(11) **EP 3 865 588 A2**
(43) Veröffentlichungstag der Anmeldung: **18.08.2021**
(21) Anmeldenummer: 21157069.2
(22) Anmeldetag: 15.02.2021
(51) Int. Cl.: C12Q 1/6841, B01L 3/00

(54) **VERFAHREN ZUM NACHWEIS VON MIKROORGANISMEN UND EIN FLUIDISCHES KANALSYSTEM**

(30) Priorität: 14.02.2020 DE 102020103957; 12.02.2021 US 202117174672
(71) Anmelder: Testo bioAnalytics GmbH, 79822 Titisee-Neustadt (DE)
(72) Erfinder: VASILEUSKAYA-SCHULZ, Zinaida, 79111 Freiburg (DE); QUEHL, Paul, 37085 Göttingen (DE); BRAGA, Daniel, 79115 Freiburg (DE); RUDEN, Serge, 79822 Titisee-Neustadt (DE); RIEMER, Joel, 79874 Breitnau (DE)
(74) Vertreter: Mertzlufft-Paufler, Cornelius

(57) **Zusammenfassung**

Es wird vorgeschlagen, ein Verfahren (7) zum Nachweis von Mikroorganismen (1) in einer Probe (9) bereitzustellen, mittels Einschleusen (10) spezifischer Nukleinsäuresonden (11) in die einzelnen Mikroorganismen (1), die mit dem vorhandenen Nukleinsäurematerial der Mikroorganismen (1) reagieren (12), und anschließendem optischen Detektieren (16) der in den einzelnen Mikroorganismen (1) erzeugten Reaktionsprodukte, wobei vor dem Einschleusen (10) der Nukleinsäuresonden (11) eine Fixierung (13) der in der Probe (9) enthaltenen Mikroorganismen (1) durchgeführt wird und ein Detergens (14) der Probe (9) zugegeben wird, bevor der Fixierungsschritt (13) abgeschlossen ist.

## Beschreibung

Die Erfindung betrifft ein Verfahren zum Nachweis von Mikroorganismen in einer Probe mittels Einschleusen spezifischer Nukleinsäuresonden in die einzelnen Mikroorganismen, die mit dem vorhandenen Nukleinsäurematerial der Mikroorganismen reagieren, und anschließendem optischen Detektieren der in den einzelnen Mikroorganismen erzeugten Reaktionsprodukte, wobei zumindest während dem Einschleusen der Nukleinsäuresonden eine Fixierung der in der Probe enthaltenen Mikroorganismen durchgeführt wird und der Probe ein Detergens zugegeben wird, bevor der Fixierungsschritt abgeschlossen ist, und wobei das Einschleusen und die Fixierung bevorzugt zeitlich parallel erfolgen.

Zum Nachweis von Nukleinsäuren in einzelnen Zellen sind beispielsweise Verfahren wie In-Situ-Hybridisierung (ISH) bekannt. Dabei werden kurze synthetische Nukleinsäuresonden eingesetzt, welche über Basenpaarungen an die nachzuweisende Ziel-Sequenz binden. Die In-Situ-Hybridisierung kann zur spezifischen Detektion von DNA und/oder RNA Molekülen verwendet werden.

Eine Variante der ISH-Technologie, bei welcher die Nukleinsäuresonden fluoreszent markiert sind, ist die Fluoreszenz-In-Situ-Hybridisierung (FISH). Die Durchführung der herkömmlichen FISH-Verfahren zum spezifischen Nachweis von Mikroorganismen mit einer besonders stark ausgeprägten äußeren Hülle umfasst (kann) zusätzlich ein Konditionierungsschritt vor einer Fixierung und Permeabilisierung (umfasssen). Durch Konditionierung wird (kann) die äußere Hülle einer Zelle soweit destabilisiert (werden), dass ein Eindringen von Fixierungs- und Permeabilisierungsagenzien in die Zelle erfolgen kann. Für eine Konditionierung werden (können) Mikroorganismen typischerweise mit Enzymen wie Achromopeptidasen, N-Acetylmuramyl-Amidasen, N-Acetylmuramylalanin-Amidasen, N-O-Diacetylmuramyl-Amidasen, Glycyl-glycin-Endopeptidasen, Lyticasen, Chitinasen, Glucanasen, Cellulasen, Proteinasen behandelt (werden).

Bei den bekannten FISH-Verfahren werden also die zu untersuchenden biologischen Proben zur Vorbereitung auf die Hybridisierung zunächst fixiert und permeabilisiert. Durch Fixierung wird die augenblickliche räumliche Struktur der in der Probe enthaltenen Mikroorganismen "eingefroren". Bei der Permeabilisierung wird die Zellhülle der in der Probe enthaltenen Mikroorganismen für Nukleinsäuresonden sowie andere von außen zugegebenen Stoffen durchlässig gemacht. Die Nukleinsäuresonden, die aus einem Oligonukleotid und einem daran gebundenen Marker bestehen, können dann die Zellhülle durchdringen und sich an die Zielsequenz im Zellinneren binden. Die Fixierung stabilisiert Makromoleküle und Zellstrukturen und verhindert so die Lyse der Zellen während der Hybridisierung. Gleichzeitig permeabilisieren die Fixierungen die Zellhülle für die fluoreszenzmarkierten Oligonukleotidsondenmoleküle. Zur Permeabilisierung/Fixierung werden typischerweise Methanol, Mischungen von Alkoholen, eine niederprozentige Paraformaldehydlösung oder eine verdünnte Formaldehydlösung verwendet. Trotz dieser Maßnahmen kann bei den vorbekannten Verfahren die Durchlässigkeit der Membran nicht in ausreichender Weise hergestellt werden, so dass das Einschleusen der Nachweissonden in die Mikroorganismen nur unzureichend ausgeführt werden kann. So kann es vorkommen, dass die Zellen überfixiert sind. Das führt dazu, dass Zellhüllekomponenten sehr stark vernetzt sind, und die Zellen vollständig undurchlässig für Nukleinsäuresonden sind. Zudem kann gegebenenfalls das Zellmaterial oft vollständig aufgelöst werden, beispielsweise wenn Fixierung zu kurz war, so dass die nachzuweisenden Substanzen im gelösten Zustand nachgewiesen werden. Dies erschwert erheblich den Nachweis individueller Mikroorganismen. Problematisch ist auch, dass beim Fixieren immer wieder die Aggregation von Zellen auftritt, welche von Bakterienstamm und Wachstumsphase abhängig ist. Dies kann Quantifizierung von Bakterien erschweren.

Damit die markierten Nukleinsäuresonden mit den Nukleinsäuren in den Zellen hybridisieren können, erfolgt zusätzlich noch ein Denaturierungsschritt, so dass die zu detektierenden Nukleinsäuren und die eingesetzten Hybridisierungssonden in einzelsträngiger Form in der Probe vorliegen. Diese Denaturierung kann insbesondere bei hohen Temperaturen von etwa 90 bis 100 °C erfolgen. Um die Morphologie der Proben jedoch zu erhalten, wird die Hybridisierung typischerweise unter Einsatz von formamidhaltigen Lösungen zur Denaturierung der doppelsträngigen Nukleinsäuren in den biologischen Proben durchgeführt. Durch den Einsatz von Formamid wird zwar bei den vorbekannten Verfahren die Schmelztemperatur von doppelsträngigen Nukleinsäuren auf 65 bis 80 °C herabgesetzt. Allerdings sind formamidhaltige Hybridisierungslösungen typischerweise mit sehr langen Reaktionsdauern verbunden.

Nach dem Fixierungsschritt, gefolgt von einem Waschschritt, um die störenden Reagenzien zu entfernen, sowie Permeabilisierungs- und Denaturierungsschritten erfolgt die Hybridisierung der eingesetzten Hybridisierungssonden mit den in der Probe enthaltenen Nukleinsäuren, so dass sich sequenzkomplementäre Abschnitte finden können. Nach der Hybridisierung folgt dann normalerweise ein weiterer Waschschritt und anschließend werden die Fluoreszenzsignale der Zelle unter einem Fluoreszenzmikroskop ausgewertet. Die Auswertung kann auch zytometrisch, beispielsweise mittels Durchflusszytometrie oder Festphasenzytometrie (Solid Phase Cytometry), erfolgen.

Aufgrund der Komplexität der herkömmlichen Verfahren, bei denen ein Reagenzaustausch sowie mehrere Verfahrensschritte erforderlich sind, sind solche Verfahren für eine schnelle und einfache Analytik nicht geeignet. Zudem ist die Verwendung von Formamid, Paraformaldehyd und Methanol wegen Toxizität nicht mit einem laborlosen Einsatz vereinbar.

Vor diesem Hintergrund ist es Aufgabe der vorliegenden Erfindung, ein einfaches und schnelles Verfahren zum Nachweis von einzelnen Mikroorganismen in einer Probe bereitzustellen, das kostengünstig und außerhalb von Laborumgebungen durchführbar ist.

Erfindungsgemäß wird diese Aufgabe durch die Merkmale von Anspruch 1 gelöst. Insbesondere wird somit erfindungsgemäß zur Lösung der genannten Aufgabe bei einem Verfahren der eingangs beschriebenen Art vorgeschlagen, dass der zu untersuchenden Probe ein Detergens zugegeben wird, bevor der Fixierungsschritt abgeschlossen ist, und das Einschleusen spezifischer Nukleinsäuresonden und die Fixierung der Mikroorganismen bevorzugt zeitlich parallel erfolgen.

Von Vorteil ist dabei, dass in Anwesenheit von einem Detergens die Durchlässigkeit für die Nukleinsäuresonden durch die Zellmembranen erhöht wird, ohne dass die bakteriellen Zellwände dabei zerstört werden. Die morphologische Integrität der Zelle bleibt erhalten, so dass Makromoleküle wie DNA, RNA und Ribosomen in der Zelle verbleiben können. Unter "Fixierung" kann im Rahmen der vorliegenden Erfindung eine morphologische Fixierung, d.h. eine Behandlung verstanden werden, bei der eine weitere Veränderung des Aufbaus der Mikroorganismen verhindert wird. Dabei ist eine Immobilisierung der inneren Strukturen der Mikroorganismen, bevorzugt ohne eine Vernetzung der Bestandteile erreichbar, so dass Mikroorganismus als Partikel erhalten bleibt.

Hierzu macht sich die Erfindung zunutze, dass ein effektives Eindringen von Nukleinsäuresonden, die mit Fluoreszenzfarbstoffen oder mit größeren Enzymmolekülen markiert sind, in die Zelle ermöglicht werden kann, indem der Probe ein Detergens zugegeben wird.

Eine vorteilhafte Ausführungsform gemäß der Erfindung sieht vor, dass das Detergens in einem Hybridisierungspuffer bereitgehalten wird. Dies kann die Zugabe des Detergens bevor die Zellen fixiert sind ermöglichen, indem in dem Hybridisierungspuffer bereits beide Stoffe Detergens und ein Fixierungsmittel enthalten sind. Dadurch können die Mikroorganismen gleichzeitig mit dem Fixierungsschritt oder vor dem Fixierungsschritt permeabilisiert, d.h. zum Einschleusen der Nachweissonden durchlässig gemacht werden. Alternativ oder zusätzlich kann vorgesehen sein, dass das Detergens in einem Vorbereitungspuffer bereitgehalten wird.

Eine vorteilhafte Ausführungsform gemäß der Erfindung sieht weiterhin vor, dass das Detergens ein ionisches Detergens, vorzugsweise Cetyltrimethylammoniumbromid, Natriumdesoxycholat oder Natriumdodecylsulfat (SDS), ist. Das Detergens kann beispielsweise auch ein nicht-ionisches Detergens, vorzugsweise Triton-X-100 oder Tween 80, sein. Alternativ oder zusätzlich kann auch ein zwitterionisches Detergens, vorzugsweise CHAPS, eingesetzt werden. In einer vorteilhaften Ausführungsform beträgt die Konzentration der denaturierenden Substanz 0-10 M, bevorzugt 4,5-8 M, besonders bevorzugt 5-6 M bei Mikroorganismen ohne eines Konditionierungsschrittes bzw. 0 M bei Mikroorganismen mit einem Konditionierungsschritt.

Eine vorteilhafte Ausführungsform gemäß der Erfindung sieht vor, dass das Detergens nicht vor Beginn der Fixierung zugegeben wird. Alternativ oder zusätzlich kann die Zugabe des Detergens vor Beginn der Fixierung, beispielsweise mit der Probe oder in dem Vorbereitungspuffer, erfolgen. Durch die Zugabe des Detergens bevor der Fixierungsschritt abgeschlossen ist, kann erreichbar sein, dass anschließend nicht lysierte, individuelle Mikroorganismen nachgewiesen werden können. Die Zellbegrenzung kann durch die Fixierung des Mikroorganismus erhalten bleiben, um Innen und Außen zumindest bis zur optischen Messung zu trennen und zu verhindern, dass Bestandteile des Mikroorganismus diesen verlassen.

Eine vorteilhafte Ausführungsform gemäß der Erfindung sieht weiterhin vor, dass der Hybridisierungspuffer mindestens eine denaturierende Substanz enthält. Alternativ oder zusätzlich kann auch eine Puffersubstanz und/oder ein Fixierungsmittel in dem Hybridisierungspuffer enthalten sein. Insbesondere kann vorgesehen sein, dass die Fixierung durch die denaturierende Substanz erfolgt. Dies kann eine vorteilhafte Zusammensetzung des Hybridisierungspuffers ermöglichen, bei dem dasselbe Mittel zur Fixierung und Denaturierung verwendet werden kann.

Eine vorteilhafte Ausführungsform gemäß der Erfindung sieht vor, dass die denaturierende Substanz eine ungiftige Substanz enthält. Insbesondere ist eine ungiftige Substanz Guanidiniumchlorid und/oder Harnstoff. Es können auch andere Substanzen, wie beispielsweise Guanidiniumthiocyanat und/oder Formamid, verwendet werden. Die Verwendung von Harnstoff ist jedoch bevorzugt. Hierdurch kann sichergestellt werden, dass der Einsatz von Harnstoff anstelle vom toxischen Formamid die Anwendung des erfindungsgemäßen Verfahrens ohne Labor ermöglicht. Von Vorteil ist dabei, dass die Reagenzien in trockenform vorgelagert und anschließend im Hausmüll entsorgt werden können. In einer vorteilhaften Ausführungsform beträgt die Konzentration der denaturierenden Substanz 4-10 M, bevorzugt 4,5-8 M, besonders bevorzugt 5-6 M.

Eine vorteilhafte Ausführungsform gemäß der Erfindung sieht vor, dass der Hybridisierungspuffer als Puffersubstanz Tris(hydroxymethyl)aminomethanhydrochlorid (TRIS-HCl) enthält. Vorteilhaft ist dabei, dass die Puffersubstanz den pH-Wert des Puffers zwischen 6,5 und 9, 0, bevorzugt zwischen 6,8 und 8,9, besonders bevorzugt zwischen 7,4 und 8,7 stabilisieren kann. Alternativ oder zusätzlich kann der Puffer aus Veronal-AcetatPuffer, HEPES-, PBS-, MES-, MOPS-, Citrat-, Barbital-Acetat-, TBS-, TE-, TAE- und TBE-Puffer ausgewählt werden. In einer vorteilhaften Ausführungsform beträgt die Konzentration der Puffersubstanz 1-100 mM, bevorzugt 2-50 mM, besonders bevorzugt 5-20 mM.

Eine vorteilhafte Ausführungsform gemäß der Erfindung sieht weiterhin vor, dass der Hybridisierungspuffer mindestens ein oder mehrere Salze, vorzugsweise Natriumchlorid, enthält. Durch die Verwendung von Salz im Hybridisierungspuffer können die Doppelstrang-Hybride aus Sonde und RNA stabilisiert werden. Das bedeutet, dass es dadurch der Wirkung denaturierender Substanzen entgegengewirkt und somit auch die Hybridisierungseffizienz erhöht werden kann. Alternativ oder zusätzlich können auch andere Salze wie beispielsweise Magnesiumchlorid und/oder Kaliumchlorid enthalten sein. In einer vorteilhaften Ausführungsform beträgt die Konzentration des Salzes 700-1500 mM, bevorzugt 750-1400 mM, besonders bevorzugt 800-900 mM bei Mikroorganismen ohne eines Konditionierungsschrittes bzw. 1200-1300mM bei Mikroorganismen mit einem Konditionierungsschritt.

Zusätzlich kann der Hybridisierungspuffer mindestens einen Chelatbildner, vorzugsweise Ethylendiamintetraessigsäure (EDTA), enthalten. Somit kann der Schutz vor Nukleasen gewährleistet werden. Der Chelatbildner kann alternativ oder zusätzlich aus Bisethylendiamin (salen), Triethylentetramin (TETA), Triaminotriethylentetramin (tren), Ethylendiamin (en), Ethylendiaminotriacetat (ted), Diethylentriaminpentaacetat (DTPA), Triethylentetraminhexaacetat (TTHA), Oxalat (ox), Tartrat (tart), Citrat (cit) ausgewählt werden. In einer vorteilhaften Ausführungsform beträgt die Konzentration des Chelatbildners 0-10 mM, bevorzugt 0, 1-5 mM, besonders bevorzugt 0,5-1 mM.

Eine vorteilhafte Ausführungsform gemäß der Erfindung sieht vor, dass das Detektieren einen Schritt des Quantifizierens der Mikroorganismen mit hybridisierten Nukleinsäuresonden umfasst. Alternativ oder zusätzlich ist auch eine Einzeldetektion der Mikroorganismen mit hybridisierten Nukleinsäuresonden durchführbar. Somit kann eine absolute Quantifizierung der nachzuweisenden Organismen auf Basis einer Partikelmessung ermöglicht werden.

Eine vorteilhafte Ausführungsform gemäß der Erfindung sieht weiterhin vor, dass die Nukleinsäuresonde komplementär zu einer DNA und/oder RNA eines nachzuweisenden Mikroorganismus ist. Dabei kann die Nukleinsäuresonde aus monoProbes, dopeProbes, tetraProbes, multiProbes, Molecular Beacons und Scorpions Sonden ausgewählt werden. Vorzugsweise können die Nukleinsäuresonden als gequenchte Molecular Beacons ausgebildet werden. Dadurch sind eine höhere Fluoreszenzintensität sowie ein besseres Signal-RauschVerhältnis erreichbar, was insbesondere für eine automatisierte Anwendung vorteilhaftsein kann.

Bei der Fluoreszenz-In-Situ-Hybridisierung kann eine zu hohe Menge an fluoreszenzmarkierter Hybridisierungssonde zu erhöhter Hintergrundfluoreszenz führen. In einer vorteilhaften Ausführungsform beträgt deshalb die Konzentration der Nukleinsäuresonde 0, 05-2 µM, bevorzugt 0,1-1 µM, besonders bevorzugt 0,13 µM.

Eine vorteilhafte Ausführungsform gemäß der Erfindung sieht weiterhin vor, dass die Nukleinsäuresonde mit einem optisch detektierbaren Marker verbunden ist. Der detektierbare Marker kann insbesondere aus Fluoreszenzmarkern, Chemolumineszenzmarkern, Affinitätsmarkern und enzymatisch aktiven Gruppen ausgewählt werden. Somit ist eine optische Detektion erreichbar. Der Affinitätsmarker kann beispielsweise Affinitätsbindungspaare Biotin-Streptavidin oder Antigen-Antikörper umfassen. Der enzymatische Marker kann beispielsweise Peroxidase, vorzugsweise Meerrettich-Peroxidase, oder Phosphatase, vorzugsweise alkalischer Phosphatase, sein.

Besonders vorteilhaft ist es, wenn eventuell entstehende Hintergrundfluoreszenz bzw. unspezifische Fluoreszenz in den hier beschriebenen FISH-Verfahren reduziert oder eliminiert wird. Dadurch können insbesondere automatisierte Detektionsverfahren spezifischer oder mit einem besseren Detektionslimit agieren. Eine unspezifische Fluoreszenz kann durch verschiedene Umstände bewirkt werden. Dazu gehören auch:
- Die unvollständige Quenching-Effizienz der eingesetzten Quencher-Moleküle. Insbesondere bei hohen Konzentrationen von eingesetzten FISH-Sonden entsteht hierdurch eine hohe unspezifische Hintergrundfluoreszenz durch überschüssige (nicht an die Ziel-RNA/DNA gebundenen) FISH-Sonden.
- Die FISH-Sonden können unspezifisch an nicht Target-Sequenzen binden oder sind unvollständig geschlossen und daher nicht oder unvollständig gequencht.
Als Gegenmaßnahmen zu der o.g. Hintergrundfluoreszenz und zur signifikanten Verbesserung des Signal-Rauschverhältnisses können daher folgende Methoden angewandt werden (eine oder einer Kombination aus diesen):
- Fluoreszenzkaskade: Gemäß/analog zum Försterresonanzenergietransfer wird ein zweiter Farbstoff in das Verfahren integriert. Die Probe wird mit einer Lichtquelle einer Wellenlänge bestrahlt, durch welche der Farbstoff/Reporter der FISH-Sonden nicht fluoresziert und somit keine Hintergrundfluoreszenz erzeugt. Stattdessen wird ein zweiter Farbstoff (hier: "Fluoreszenzdonor" mit Donorfluorophor) in den Assay eingebracht, welcher durch die eingebrachte Anregungswellenlänge fluoresziert und das emittierte Licht (durch die Fluoreszenzverschiebung in einen längerwelligen Bereich) wiederum in der Lage ist, den Farbstoff der z.B. FISH-Sonden / Molecular Beacons (hier: "Fluoreszenzakzeptor" mit Akzeptorfluorophor) zur Fluoreszenz anzuregen. Das Emissionsspektrum in welchem das Donorfluorophor fluoresziert, liegt dabei außerhalb oder nur in geringen Teilen innerhalb des Detektionsspektrums / der Detektionswellenlängen des Detektors und erzeugt daher keine relevante Hintergrundfluoreszenz. Der Mechanismus dieser zweistufigen Fluoreszenz funktioniert nur bzw. nur ausreichend, wenn Donor- und Akzeptorfluorophor in ausreichender räumlicher Nähe sind und nicht durch ein Quenchermolekül gequencht sind. Eingesetzte FISH-Sonden können bei diesem Verfahren die Fluoreszenzakzeptor oder die Fluoreszenzdonor-Funktion oder auch beide Funktionen übernehmen. Ideal geeignet sind hierbei Kombinationen aus jeweils mit dem Donor- und Akzeptorfluorophor versehene Oligos (DNA/RNA-Moleküle), welche in unmittelbare Nähe zueinander gebracht werden. Hierzu werden aneinandergrenzende oder durch Sekundärstrukturen nah beieinander liegende Bindestellen innerhalb des Zielorganismus gewählt, an welchen die Oligos (Fluoreszenzdonor und -Akzeptor) binden. Das Akzeptor- oder Donorfluorophor kann jedoch auch ein z.B. profluoreszenter Farbstoff sein und durch Enzyme innerhalb der Zielzellen zu einem für den Nachweis nutzbaren Fluorophor umgesetzt werden (z.B. Carboxyfluorescein) oder sich in bestimmten Zellstukturen (wie RNA, DNA, Proteinen und Lipiden) anlagern (Farbstoffe wie z.B. SYBR green, Ethidiumbromid, Coomassie) oder sich innerhalb des Zielorganismus akkumulieren (z.B. Tetramethylrhodamin-methylester) und somit ebenfalls in ausreichende räumliche Nähe des Donor- bzw. Akzeptorfluorophors gebracht werden. Besonders vorteilhaft können dabei Verfahren sein, welche entweder den Donor- oder den Akzeptorfluorophor mit einem unspezifischen Target (das Target kann z.B. RNA- oder DNA sein) in ausreichend Nähe des jeweils anderen bringen, da so Kosten für die spezifische Synthese von z.B. spezifischen FISH-Sonden vermieden werden können. Gleichzeitig lässt sich das Verfahren so standardisiert für die Verbesserung von anderen FISH-Assays und unabhängig von deren spezifischen Zielsequenzen verwenden. Insbesondere der Einsatz von Fluorophorgelabelten "random" Oligos, wie z.B. "random Hexamers", oder anderen zufälligen Oligo-Sequenzen ist dabei vorteilhaft, da eine Mischung aller möglichen (z.B.) Oligo-Hexamer-Sequenzoptionen (dies ist die Bedeutung von "random Hexamers") an allen einzelsträngigen Sequenzoptionen von Nukleinsäuren binden können. Diese "random" Oligos können sowohl am 3' und/oder am 5'-Ende mit einem oder mehreren Farbstoffen gelabelt sein. Durch die Nutzung einer Kombination von Fluoreszenz-Donoren- und -Akzeptoren ist es außerdem möglich, generell auf den Einsatz von Quenchern beim hier beschriebenen FISH-Verfahren zu verzichten und so kostengünstigere FISH-Sonden (ohne Quenchermoleküle) und bei gleicher Spezifität zu erstellen, da die Spezifität durch die notwendige räumliche Nähe der gebundener Farbstoffe (z.B. zwei spezifische Oligos mit Farbstoffen) erreicht werden kann. Für die Nutzung von unspezifischen Fluoreszenzdonoren oder -Akzeptoren ist darauf zu achten, dass diese erst nach dem Annealingschritt ("Bindungsschritt") mit dem spezifischen Reagenz (spezifische FISH-Sonde) eingebracht werden, da diese sonst ggf. die Bindungspositionen der spezifischen FISH-Sonden besetzen und zu falsch-negativen Ergebnissen führen. Werden profluoreszierende Farbstoffe eingesetzt, welche zunächst z.B. durch Enzyme der angefärbten Zellen umgesetzt werden müssen oder sich nur in Zellen mit einer ausreichend intakten Zellemembran akkumulieren, so lassen sich daraus zusätzlich Schlussfolgerung hinsichtlich der Vitalität der zu markierenden Zielorganismen treffen.
- Quenching-Sonden nach Annealing-Schritt: Falsch-gebundene oder unzureichend geschlossene FISH-Sonden ("molecular beacons") können erneut oder mit besserer Effizienz gequencht werden. Hierzu werden (können) nach dem Annealingschritt der FISH-Sonden weitere Oligos eingebracht (werden), welche komplementär zu den eingesetzten FISH-Sonden sind. Diese Oligos (hier: "Quencher-Oligos") tragen (können) ein oder mehrere Quenchermoleküle an deren Enden (tragen) und binden (können) an den FISH-Sonden (binden). Die komplementäre Sequenz ist (kann) dabei länger als die hairpin-bildende Halssequenz der FISH-Sonden (sein) und führen daher nach deren Annealing ("Bindung") an den FISH-Sonden zu einer stabilen linearisierten (zweisträngigen) Struktur. In dieser Struktur liegen (können) die Fluorophore der FISH-Sonden nun durch den Quencher der "Quencher Oligos" gequencht vor(liegen) und erzeugen (können) nur noch eine geringe Hintergrundfluoreszenz (erzeugen).
- Immobilisierung von freien FISH-Sonden vor Messung: Überschüssige FISH-Sonden können aus dem Probenansatz entfernt werden. Hierzu wird (kann) das Probengemenge, vor der Messung und nach dem Annealingschritt der FISH-Sonden an die nachzuweisenden Zielmoleküle, über eine Oberfläche geführt, welche die überschüssigen (nicht an Zielsequenzen gebundene FISH-Sonden) bindet (binden kann) und vor einer Messung der Probe aus dem Ansatz entfernt (werden). Alternativ können auch Körper (wie z.B. "beads" / Kügelchen) zu der Probe hinzugegeben werden, an welchen die überschüssigen FISH-Sonden binden. Anschließend werden diese Körper mitsamt der überschüssigen FISH-Sonden aus dem Reaktionsansatz abgeschieden. Die Oberflächen bzw. Körper sind (können) dabei in beiden Fällen funktionalisiert (werden). Entweder werden sie dabei mit zu den FISH-Sonden komplementären Oligos (z.B. DNA- oder RNA-Fragmente) oder mit anderen Hilfsmitteln (z.B. gegen die Fluorophore oder Quencher der FISH-Sonden gerichteten Antikörpern) beschichtet/funktionalisiert. Typischerweise können hierzu biotinylierte komplementäre Oligos eingesetzt werden, welche an eine Strepatividinbeschichte Oberfläche gebunden sind. Ebenfalls ist es möglich die eingesetzten FISH-Sonden schon von Beginn an mit möglichen Bindungshilftsmittel (wie z.B. Biotin oder Streptavidin) zu koppeln und die zur Immobilisierung eingesetzten Oberflächen/Körper mit einem komplementären Bindungsmittel zu funktionalisiern (z.B. FISH-Sonden mit Biotin gekoppelt und Oberflächen mit Streptavidin beschichtet). Die Probe wird/kann (mehrere Male) über eine derartig funktionalisierte Oberfläche/Körper gespült und anschließend wieder entnommen/entnommen werden. Ein großer Teil der zuvor nicht-gebundenen FISH-Sonden wird (kann) dabei auf dieser Oberfläche / diesem Körper immobilisiert und somit die aus den überschüssigen FISH-Sonden entstehen Hintergrundfluoreszenz aus dem Verfahren entfernt (werden).
- Fluoreszenzzerstörung überschüssiger FISH-Sonden vor Messung: Die Fluorophore der überschüssigen (nicht an die Ziel-RNA/DNA gebundenen) FISH-Sonden können physikalisch, chemisch oder biologisch so verändert werden, dass sie keine für die Messung relevante Fluoreszenz aufweisen. Dies kann z.B. durch die Zugabe von Reagenzien (z.B. Enzyme) erreicht werden (z.B. P450-Monooxygenasen), welche z.B. aromatische Strukturen der Fluorophoren modifizieren (z.B. hydroxylieren) und somit die relevanten Fluoreszenzeigenschaften verändern oder unterbinden. Derartige Reagenzien werden (können) dabei erst nach dem Annealingschritt der FISH-Sonden hinzugeben (werden). Die Methode wird (kann) dabei so gewählt (werden), dass die Fluorophore der innerhalb der Zielorganismen gebundenen FISH-Sonden nicht betroffen sind, da sie z.B. durch die Zellmembran des Zielorganismus geschützt sind und so das Fluoreszenzinhibierende Reagenz nicht in deren Nähe gelangen bzw. nicht mit diesen interagieren kann.
- Reduktion des fluoreszierenden Hintergrundes durch den Einsatz von "freien" Quencher-Molekülen in Konzentrationen größer als 1mM. Die hohe Konzentration an freien Quencher-Molekülen bewirkt (kann) eine präferierte Löschung bzw. Reduktion der Fluoreszenz von freien/überschüssigen Oligosonden (z.B. linear, molekular beacon, scorpions etc.) außerhalb der markierten Mikroorganismen (bewirken). Die Löschung der intrazellulär gebundenen Fluoreszenz-Sonden durch freie Quencher-Moleküle wird (kann) durch die Tatsache vermindert (werden), dass die Diffusion der freien Quencher-Moleküle in die Zelle und die Verteilung innerhalb der Zelle durch die Zellbestandteile erschwert bzw. verhindert wird. Die Auswahl der Quencher-Moleküle hängt (kann) vom zu quenchenden Farbstoff ab(hängen). Für die Farbstoffe FAM, Alexa488, Atto488 u.Ä. eignen sich z.B. die Isomere von Methylrot (para-Methylrot, meta-Methylrot, o-Methylrot; 4- bzw.3- bzw. 2-{[4-(Dimethylamino)phenyl]diazenyl}benzoesäure).
- Immobilisierung der Zielorganismen: Die Zielorganismen können in einer besonders vorteilhaften Ausgestaltung des Messsystems (z.B. einem "Lab on a Chip"-System) auf Strukturen wie Filtern und insbesondere Track Etched Membranes zurückgehalten und/oder aufkonzentriert werden. Somit ist es möglich die Zielorganismen vom restlichen Probenansatz abzuscheiden und die Zielorganismen zusätzlich mit Spülsubstanzen von überschüssiger FISH-Sonden zu befreien, während markierte Zielorganismen zurückgehalten werden. Das hier beschriebene FISH-Verfahren kann dabei auch derartig ausgestaltet sein, dass die beschriebenen Reaktionsschritte, durch die sequentielle Zugabe aller benötigten Reagenzien, direkt auf der Filterstruktur (z.B. Track Etched Membran), durchgeführt werden. Hierdurch können auch die eingesetzten Reagenzien in Ihrer Stoffmenge reduziert und somit Herstellungskosten eingespart werden. Dazu ist es notwendig die Reagenzien einzeln auf die Filterstruktur zu spülen (z.B. durch pneumatischen und/oder zentrifugalen Transport). Besonders vorteilhaft sind Auslegungsformen, in denen die Zielorganismen zunächst auf eine Filterstruktur aufgespült und anschließend (innerhalb des mikrofluidischen Systems) wieder eluiert werden können, z.B. durch Eluieren/Abspülen mit einer Flüssigkeit entgegen der Aufspülrichtung / von der den Zielorganismen abgewandten Filterseite aus. Die Filterstruktur sollte dabei in ihren Eigenschaften (wie Eigenfluoreszenz) so ausgelegt sein, dass auch eine direkte Auslesung des Messergebnisses auf der Filterstruktur erfolgen kann.
- Metering innerhalb des fluidischen Systems: Das hier beschriebene FISH-Verfahren gestattet es breite Abweichungen eingebrachter Probenvolumina auszugleichen / abzupuffern. Hierzu ist es notwendig/vorteilhaft bestimmte Reagenzien und insbesondere die FISH-Sonden in hohem Überschuss zuzusetzen, um auch bei unerwartet hohen Probenvolumina über eine ausreichend hohe Konzentration von FISH-Sonden zur verfügen. Ein hoher Überschuss der FISH-Sonden zeichnet (kann) sich jedoch auch durch eine hohe Hintergrundfluoreszenz aus(zeichnen). Ziel sollte es daher sein, mit der minimalen und exakt abgestimmten Konzentration FISH-Sonden arbeiten zu können. Hierzu wird (kann) auf einer fluidischen Plattform zunächst ein Meteringschritt durchgeführt (werden), welcher das Ausgangsprobenvolumen verlustfrei und per z.B. Zentrifugalkraft standardisiert ("metert"). Durch das nun bekannte Ausgangsvolumen können die FISH-Sonden und restlichen Reagenzien exakt in ihrer Konzentration eingestellt und somit innerhalb ihres Perfomance-Optimums verwendet werden. Hierzu wird zunächst die zu untersuchenden Probe z.B. per Zentrifugalkraft eingebracht und anschließend mit einem Überschuss einer Pufferflüssigkeit bis zu einem Überlaufkanal aufgefüllt. Nicht benötigte Pufferflüssigkeit wird über einen Überlaufkanal abgeführt. Die Position des Überlaufkanals ist dabei so gewählt, dass das Flüssigkeitsvolumen bis zum Überlaufen dieser "Meteringkammer" bekannt ist. Durch das Design der "Meteringkammer" ist dabei sichergestellt, dass kein Probenmaterial verlorengeht.

Das hier beschrieben FISH-Verfahren gestattet (kann) Aussagen über die Vitalität von untersuchenden Organismen anhand derer rRNA-Konzentration (gestatten). Ziel ist es die Unterscheidung zwischen "lebenden" und "toten" Mikroorganismen schnell und sehr spezifisch zu erreichen. Das hier beschrieben FISH-Verfahren basiert generell auf dem Abbau von rRNA toter Mikroorganismen bzw. einem Aufbau von rRNA von lebenden Mikroorganismen zur Unterscheidung der Vitalität der Mikroorganismen. Die unterschiedliche Membran-Permeabilität von lebenden und toten Organismen kann jedoch ebenfalls genutzt werden, um lebende und tote Organismen schneller voneinander zu unterscheiden. Annahme dabei ist, das tote Zellen eine deutlich erhöhte Permeabilität der Zellmembran aufweisen. Mit den folgenden Optionen ist es möglich die Detektionsschwelle für "lebende" Mikroorganismen herabzusetzen, da toten Mikroorganismen nicht mehr (ausreichend) markiert werden und lebende Mikroorganismen (für deren Signalverstärkung bzw. Erhöhung des Unterschieds zwischen lebenden und toten Mikroorganismen) keine zusätzliche Nukleinsäuren (z.B. rRNA) für die Unterscheidung lebend/tot aufbauen müssen. Somit kann das Verfahren signifikant beschleunigt werden:
- Target Depletion: Die Ziel-Nukleinsäuren (wie rRNA) können vor dem eigentlichen Verfahren in toten Mikroorganismen abgebaut werden. Somit ist es möglich die Detektionsschwelle für "lebende" Mikroorganismen herabzusetzen und weniger rRNA-Aufbau (Zeit) dieser abzuwarten, da tote Mikroorganismen, durch die ihnen fehlenden Zielsequenzen nicht mehr oder kaum noch durch das Verfahren markiert werden können. Hierzu können entweder Ribonukleasen (wie Ribonuklease A) allein zugesetzt werden, oder Kombinationen aus Ribonukleasen (z.B. Ribonuklease H) mit Nukleinsäuren (z.B: DNA-Oligos). Im ersten Fall baut Ribonuklease A die ihr zugänglichen Gesamt-RNA ab. Im zweiten Fall binden die eingebrachten Nukleinsäuren spezifisch an die abzubauenden Nukleinsäuren (z.B. rRNA) und die Ribonuklease H erkennt den Heteroduplex aus eingebrachter DNA und der Ziel-rRNA. Dieser Heteroduplex wird nun durch die Ribonuklease H spezifisch abgebaut. Den Ribonukleasen und Nukleinsäuren ist es dabei nicht möglich die Membranen lebender Mikroorganismen zu durchdringen - somit werden lediglich Nukleinsäuren toter Organismen mit ausreichend permeablen Zellmembranen abgebaut. Hierzu können auch weitere Strukturen ("Ankerstrukturen") an z.B. die DNA-Oligos synthetisiert werden, welche die Durchdringung intakter Zellmembranen weiter erschweren. Generell ist bei diesem Vorgehen darauf zu achten, dass die eingebrachten Ribonukleasen vor Zugabe der FISH-Sonden entweder inaktiviert, gehemmt oder aus dem Probenansatz (z.B. durch Spülschritte auf einem Filter / track etched membran) entfernt werden. Zusätzlich kann diese Methode auch mit Detektionsverfahren kombiniert werden, welche z.B. auf dem Försterresonanzenergietransfer basieren oder das Auslesen mehrere Markierungen in einem Mikroorganismus ermöglichen. So können beispielsweise Proteine oder DNA als Fluoreszenzdonor (oder -Akzeptor) oder als zweite Zielstruktur für Markierungen verwendet werden und vor Beginn des eigentlichen Verfahrens bei toten Zellen abgebaut werden (z.B. durch Proteinasen oder Desoxyribonuklease, welche intakte Membranen lebender Organismen nicht durchdringen können). Somit werden lediglich lebende Mikroorganismen durch das Verfahren erfasst, da toten Zellen entweder über keinen Fluoreszenzdonor (oder -Akzeptor) und somit über keine z.B. Fluoreszenz im relevanten Emissionsbereich verfügen, oder lediglich über die Fluoreszenz der FISH-Sonden, nicht aber über die Fluoreszenz des Lebend/Tot-Indikators (z.B. nicht mehr vorhandene DNA oder Proteine) und somit lediglich einfach-markiert sind.
- Target Blocking: Die unterschiedliche Membran-Permeabilität von lebenden und toten Mikroorganismen kann genutzt werden, um Nukleinsäure (z.B. DNA-Oligos) in tote Mikroorganismen einzubringen und damit deren Untersuchungs-relevante Bindungsstellen (z.B. in deren rRNA) zu besetzen. Anschließend können z.B. FISH-Sonden nicht mehr an diesen Positionen binden, da die Stellen bereits besetzt sind. Es ist darauf zu achten, dass die eingebrachten Oligos zur Besetzung der relevanten Bindungsstellen, vor Einbringung der FISH-Sonden bzw. vor Permeabilisierung der lebenden Mikroorganismen, aus dem Probenansatz entfernt werden. Dies kann beispielsweise durch das Spülen des Probenansatzes über einen Filter (z.B. Track Etched Membran) erfolgen.

Einsatz von Lebend/Tot-Färbungen: Es ist möglich das hier verwendeten FISH-Verfahren mit einer zusätzlichen lebend/tot-Unterscheidung der relevanten Mikroorganismen durchzuführen. Hierzu können die Zielorganismen auf einem Filter (wie einer track etched Membran) fixiert und mit einem Lebend/Tot-Farbstoff (wie z.B. Propidiumiodid) behandelt werden. Die Membran wird durch einen Sensor kartiert und für die jeweiligen Mikroorganismen der Zustand "lebend" oder "tot" festgehalten. Anschließend oder gleichzeitig wird das FISH-Verfahren durchgeführt und die durch die FISH-Sonden positivmarkierten Mikroorganismen zusätzlich mit dem Zustand "lebend- " oder "tot-" in der Datenerfassung versehen. Darüber hinaus können die Mikroorganismen auch mit dem hier beschriebenen FISH-Verfahren markiert und zusätzlich mit einem Lebend- oder Tot-Farbstoff (z.B. Propidiumiodid) versehen werden, falls dieser über andere spektrale Eigenschaften als die Farbstoffe der FISH-Sonden verfügt. Anschließend werden (können) je Zielorganismus mehrere spektrale Eigenschaften (z.B. Fluoreszenzen in verschiedenen Wellenlängen bzw. Spektren) ausgelesen und Informationen zu z.B. Organismusart und deren Vitalität gleichzeitig aufgenommen (werden).

Eine vorteilhafte Ausführungsform gemäß der Erfindung sieht vor, dass das Verfahren mit einem fluidischen Kanalsystem ausgeführt werden kann, insbesondere mit einem scheibenförmigen Probenträger. Von Vorteil ist dabei, dass ein spezifischer Nachweis von Mikroorganismen in unterschiedlichen Applikationsfeldern ermöglicht werden kann. Beispielsweise kann das erfindungsgemäße Verfahren zur mikrobiologischen Lebensmittelkontrolle, Hygienekontrolle, klinischen und biotechnologischen Anwendungen sowie Umweltanalyse zum Einsatz kommen.

Eine bevorzugte Anwendung sieht ein fluidisches Kanalsystem vor, mit Mitteln zum Durchführen des Verfahrens, insbesondere wie zuvor beschrieben und/oder nach einem der auf ein Verfahren gerichteten Ansprüche. Beispielsweise können in dem fluidischen Kanalsystem zum Durchführen des erfindungsgemäßen Verfahrens ein Detektionsbereich und ein Vorbereitungsbereich ausgebildet sein. Insbesondere kann vorgesehen sein, dass die Querschnitte der Kanäle des fluidischen Kanalsystems auf Abmessungen der Mikroorganismen angepasst sein können.

Das fluidische Kanalsystem kann beispielsweise in Form eines Probenträgers ausgebildet sein. Der erfindungsgemäße Probenträger umfasst insbesondere mindestens eine Kavität mit einer Nukleinsäuresonde und mindestens einem Detergens. Alternativ oder zusätzlich kann der Probenträger Mittel zur optischen Zählung von markierten Mikroorganismen umfassen.

Der erfindungsgemäße Probenträger kann als ein scheibenförmiger Probenträger ausgebildet sein. Beispielsweise kann der Probenträger als ein flacher Probenträger ausgebildet sein. Von Vorteil ist dabei, dass durch die Scheibenform des Probenträgers die Zentrifugalkraft für die Fluidförderung ausgenutzt werden kann. Die Fluidförderung ist auch über Druck oder auf eine andere Weise erreichbar. Alternativ kann der Probenträger eine dreidimensionale Erstreckung, beispielsweise in Form eines Zylinders oder küvettenartig, aufweisen.

Beispielsweise kann die Scheibenförmigkeit rotationssymmetrisch ausgebildet sein. Dies kann für ein Zentrifugieren von Vorteil sein. Es sind auch alternativ rechteckförmige Probenträger wie bei einer Chipkarte oder segmentförmige Probenträger wie bei einem Pizzastück bildbar.

Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:
- Fig. 1: ein herkömmliches, Fluoreszenz-In-Situ-Hybridisierung (FISH)-basiertes Verfahren in einer schematischen Darstellung für Mikroorganismen ohne einer stark ausgeprägten äußeren Hülle,
- Fig. 2: ein erfindungsgemäßes FISH-Verfahren in einer schematischen Darstellung für Mikroorganismen ohne einer stark ausgeprägten äußeren Hülle,
- Fig. 3: das Verfahren gemäß Fig. 2 in einer detaillierten Darstellung für Mikroorganismen ohne einer stark ausgeprägten äußeren Hülle,
- Fig. 4: ein herkömmliches, Fluoreszenz-In-Situ-Hybridisierung (FISH)-basiertes Verfahren in einer schematischen Darstellung für Mikroorganismen mit einer stark ausgeprägten äußeren Hülle,
- Fig. 5: ein erfindungsgemäßes FISH-Verfahren in einer schematischen Darstellung für Mikroorganismen mit einer stark ausgeprägten äußeren Hülle,
- Fig. 6: das Verfahren gemäß Fig. 2 in einer detaillierten Darstellung für Mikroorganismen mit einer stark ausgeprägten äußeren Hülle.

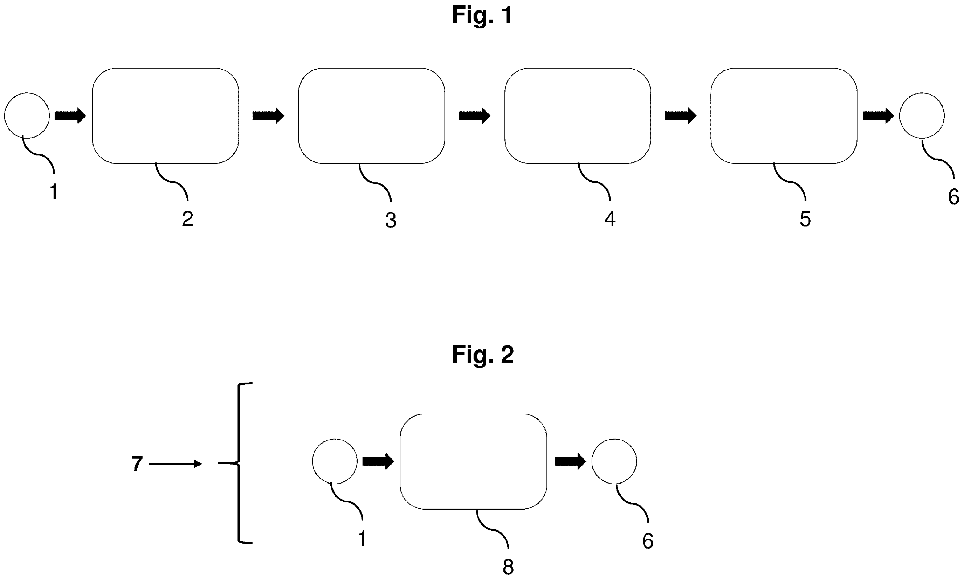

In den Figuren 1 bis 3 ist ein Verfahren zum Nachweis von Mikroorganismen ohne einer stark ausgeprägten äußeren Hülle in verschiedenen Ausführungen gezeigt.

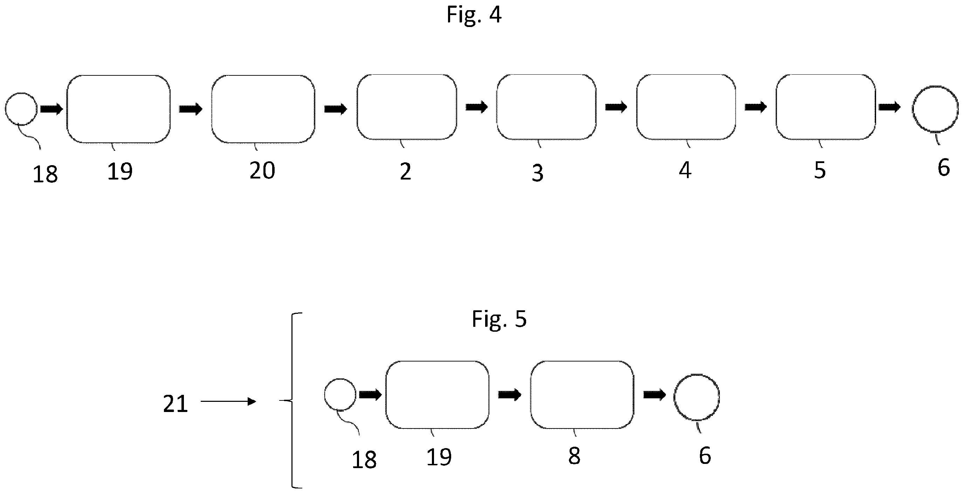

In den Figuren 4 bis 6 ist ein Verfahren zum Nachweis von Mikroorganismen mit einer stark ausgeprägten äußeren Hülle in verschiedenen Ausführungen gezeigt.

Fig. 1 zeigt ein herkömmliches FISH-Verfahren zum spezifischen Nachweis von Nukleinsäuren in einzelnen Mikroorganismen 1, umfassend die folgenden Schritte: Fixierung und Permeabilisierung 2 der in einer Probe enthaltenen Mikroorganismen 1; Waschen 3, um die zur Permeabilisierung verwendeten Reagenzien zu entfernen; Inkubation der fixierten und permeabilisierten Mikroorganismen 1 mit Nukleinsäuresonden, um eine Hybridisierung 4 herbeizuführen; Entfernen bzw. Abwaschen 5 der nicht hybridisierten Nukleinsäuresonden; und anschließende Analyse 6 der mit den Nukleinsäuresonden hybridisierten Mikroorganismen 1.

Fig. 2 zeigt ein erfindungsgemäßes Verfahren 7 zum spezifischen Nachweis von Mikroorganismen 1, welches die Schritte der Permeabilisierung, Fixierung und Hybridisierung in einen einzigen Verfahrensschritt 8 zusammenfasst. Es werden keine zusätzlichen Waschschritte oder Pufferaustausche benötigt.

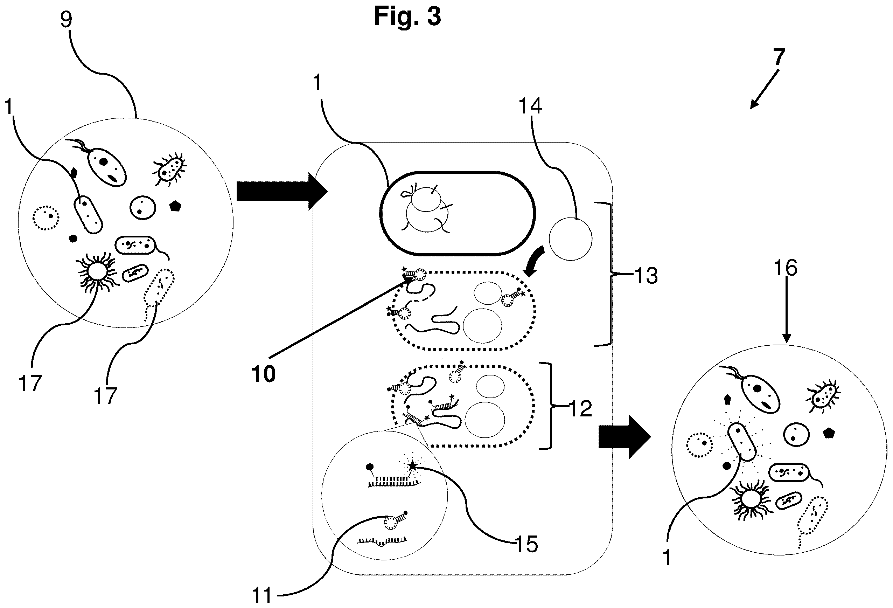

Fig. 3 zeigt ein erfindungsgemäßes Verfahren 7 zum Nachweis von Mikroorganismen 1 in einer Probe 9 mittels Einschleusen 10 spezifischer Nukleinsäuresonden 11 in die einzelnen Mikroorganismen 1, wobei die Nukleinsäuresonden 11 mit dem vorhandenen Nukleinsäurematerial der Mikroorganismen 1 reagieren. Es findet somit eine Hybridisierung 12 statt.

Aus Fig. 3 ist weiterhin ersichtlich, dass zumindest während dem Einschleusen 10 der Nukleinsäuresonden 11 eine Fixierung 13 der in der Probe 9 enthaltenen Mikroorganismen 1 durchgeführt wird, wodurch eine weitere Veränderung des Aufbaus der Mikroorganismen verhindert wird. Es ist bevorzugt, dass das Einschleusen 10 und die Fixierung 13 zeitlich parallel erfolgen.

Um ein effektives Eindringen von Nukleinsäuresonden 11 in die Zellen zu ermöglichen, ohne dass die bakteriellen Zellwände dabei zerstört werden und so die morphologische Integrität der Zellen erhalten bleibt, wird der Probe 9 ein Detergens 14 zugegeben, bevor der Fixierungsschritt 13 abgeschlossen ist (vgl. Fig. 3). Somit ist eine absolute Quantifizierung der nachzuweisenden Organismen auf Basis einer Partikelmessung erreichbar.

Wie aus Fig. 3 weiterhin erkennbar ist, ist die Nukleinsäuresonde 11 mit einem optisch detektierbaren Marker 15 verbunden, um anschließend das optische Detektieren 16 der in den einzelnen Mikroorganismen 1 erzeugten Reaktionsprodukte zu ermöglichen.

Bei einer bevorzugten Anwendung wird zum spezifischen Nachweis von Mikroorganismen ohne einer stark ausgeprägten äußeren Hülle, eine Mikroorganismen-haltige Probe mit einem Hybridisierungspuffer (900 mM NaCI, 20 mM Tris/HCl, 0,01 % SDS, 5,3 M Harnstoff, 1 mM EDTA, 0,13 µM Hybridisierungssonde und pH 8,0) versetzt und für einen Zeitraum von 15 bis 90 Minuten bei einer Temperatur von 52°C inkubiert. Nach Ende dieser Inkubationszeit werden die fertig hybridisierten Proben zytometrisch oder Fluoreszenz-mikroskopisch analysiert.

Erfindungsgemäß wird somit vorgeschlagen, ein Verfahren 7 zum Nachweis von Mikroorganismen 1 in einer Probe 9 bereitzustellen, mittels Einschleusen 10 spezifischer Nukleinsäuresonden 11 in die einzelnen Mikroorganismen 1, die mit dem vorhandenen Nukleinsäurematerial der Mikroorganismen 1 reagieren 12, und anschließendem optischen Detektieren 16 der in den einzelnen Mikroorganismen 1 erzeugten Reaktionsprodukte, wobei vor dem Einschleusen 10 der Nukleinsäuresonden 11 eine Fixierung 13 der in der Probe 9 enthaltenen Mikroorganismen 1 durchgeführt wird und ein Detergens 14 der Probe 9 zugegeben wird, bevor der Fixierungsschritt 13 abgeschlossen ist.

Fig. 4 zeigt ein herkömmliches FISH-Verfahren zum spezifischen Nachweis von Nukleinsäuren in einzelnen Mikroorganismen mit einer stark ausgeprägten äußeren Hülle 18, umfassend die folgenden Schritte: enzymatische Konditionierung 19 der in einer Probe enthaltenen Mikroorganismen 18 ; Waschen 20, um die zur Konditionierung verwendeten Reagenzien zu entfernen; Fixierung und Permeabilisierung 2 der in einer Probe enthaltenen Mikroorganismen 18; Waschen 3, um die zur Permeabilisierung verwendeten Reagenzien zu entfernen; Inkubation der konditionierten, fixierten und permeabilisierten Mikroorganismen 18 mit Nukleinsäuresonden, um eine Hybridisierung herbeizuführen; Entfernen bzw. Abwaschen 5 der nicht hybridisierten Nukleinsäuresonden; und anschließende Analyse 6 der mit den Nukleinsäuresonden hybridisierten Mikroorganismen 18.

Fig. 5 zeigt ein erfindungsgemäßes Verfahren 21 zum spezifischen Nachweis von Mikroorganismen mit einer stark ausgeprägten äußeren Hülle 18, welches die Schritte der Konditionierung 19, Permeabilisierung, Fixierung und Hybridisierung beinhaltet jedoch die Schritte der Permeabilisierung, Fixierung und Hybridisierung in einen einzigen Verfahrensschritt 8 zusammenfasst. Es werden keine zusätzlichen Waschschritte oder Pufferaustausche benötigt.

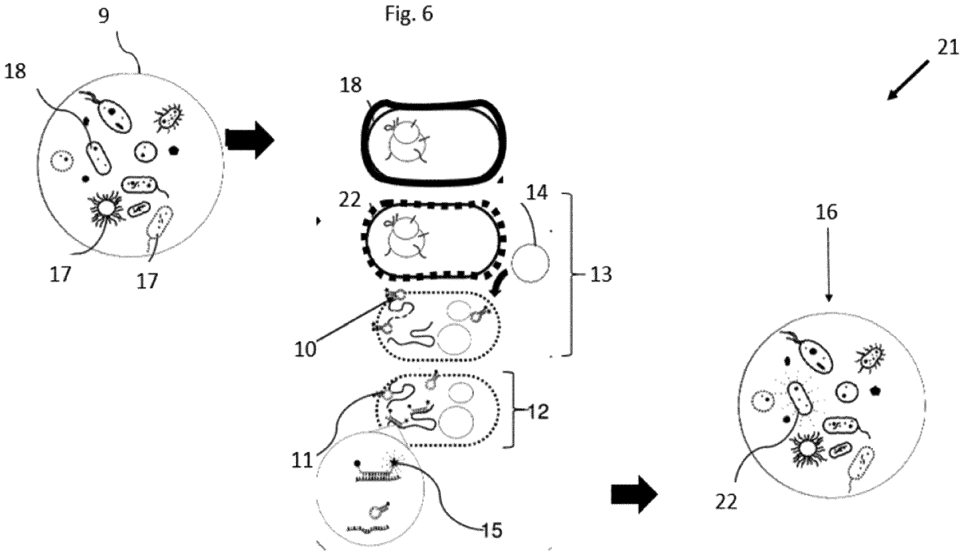

Fig. 6 zeigt ein erfindungsgemäßes Verfahren 21 zum Nachweis von Mikroorganismen mit einer stark ausgeprägten äußeren Hülle 18 in einer Probe 9 mittels Einschleusen 10 spezifischer Nukleinsäuresonden 11 in die einzelnen konditionierten Mikroorganismen 22, wobei die Nukleinsäuresonden 11 mit dem vorhandenen Nukleinsäurematerial der konditionierten Mikroorganismen 22 reagieren. Es findet somit eine Hybridisierung 12 statt.

Aus Fig. 6 ist weiterhin ersichtlich, dass zumindest während dem Einschleusen 10 der Nukleinsäuresonden 11 eine Fixierung 13 der in der Probe 9 enthaltenen und konditionierten Mikroorganismen 22 durchgeführt wird, wodurch eine weitere Veränderung des Aufbaus der Mikroorganismen verhindert wird. Es ist bevorzugt, dass das Einschleusen 10 und die Fixierung 13 zeitlich parallel erfolgen.

Um ein effektives Eindringen von Nukleinsäuresonden 11 in die Zellen zu ermöglichen, ohne dass die bakteriellen Zellwände dabei vollständig zerstört werden und so die Integrität der Zellen erhalten bleibt, wird der Probe 9 nach der Konditionierung ein Detergens 14 zugegeben, bevor der Fixierungsschritt 13 abgeschlossen ist (vgl. Fig. 6). Somit ist eine absolute Quantifizierung der nachzuweisenden Organismen auf Basis einer Partikelmessung erreichbar.

Wie aus Fig. 6 weiterhin erkennbar ist, ist die Nukleinsäuresonde 11 mit einem optisch detektierbaren Marker 15 verbunden, um anschließend das optische Detektieren 16 der in den einzelnen konditionierten Mikroorganismen 22 erzeugten Reaktionsprodukte zu ermöglichen.

Bei einer bevorzugten Anwendung wird zum spezifischen Nachweis von Mikroorganismen mit einer stark ausgeprägten äußeren Hülle, eine Mikroorganismen-haltige Probe mit einem und/oder mehreren Enzymen zwecks Konditionierung für 10 bis 30 Minuten bei einer Temperatur von 52°C inkubiert. Direkt im Anschluss wird die Probe mit einem Hybridisierungspuffer (1250 mM NaCI, 20 mM Tris/HCl, 0,01 % SDS, 1 mM EDTA, 0,13 µM Hybridisierungssonde und pH 8,0) versetzt und für einen Zeitraum von 20 bis 90 Minuten bei einer Temperatur von 52°C inkubiert. Nach Ende dieser Inkubationszeit werden die fertig hybridisierten Proben zytometrisch oder Fluoreszenz-mikroskopisch analysiert.

Erfindungsgemäß wird somit vorgeschlagen, ein Verfahren 21 zum Nachweis von von Mikroorganismen mit einer stark ausgeprägten äußeren Hülle 18 in einer Probe 9 bereitzustellen, mittels Einschleusen 10 spezifischer Nukleinsäuresonden 11 in die einzelnen vorab konditionierten Mikroorganismen 22, die mit dem vorhandenen Nukleinsäurematerial der Mikroorganismen 22 reagieren 12, und anschließendem optischen Detektieren 16 der in den einzelnen Mikroorganismen 22 erzeugten Reaktionsprodukte, wobei vor dem Einschleusen 10 der Nukleinsäuresonden 11 eine Fixierung 13 der in der Probe 9 enthaltenen Mikroorganismen 18 durchgeführt wird und ein Detergens 14 der Probe 9 zugegeben wird, bevor der Fixierungsschritt 13 abgeschlossen ist.

### Bezugszeichenliste

- 1: nachzuweisende Mikroorganismen ohne einer stark ausgeprägten äußeren Hülle
- 2: Fixierung und Permeabilisierung gemäß herkömmlichem FISH-Verfahren
- 3: Waschen gemäß herkömmlichem FISH-Verfahren
- 4: Hybridisierung gemäß herkömmlichem FISH-Verfahren
- 5: Waschen gemäß herkömmlichem FISH-Verfahren
- 6: Analyse
- 7: erfindungsgemäßes Verfahren für Mikroorganismen ohne einer stark ausgeprägten äußeren Hülle
- 8: Permeabilisierung, Fixierung und Hybridisierung, umfasst in einem Verfahrensschritt
- 9: Probe mit Mikroorganismen
- 10: Einschleusen spezifischer Nukleinsäuresonden
- 11: Nukleinsäuresonde
- 12: Hybridisierung
- 13: Fixierung der Mikroorganismen
- 14: Detergens
- 15: Marker
- 16: optisches Detektieren für Mikroorganismen ohne einer stark ausgeprägten äußeren Hülle
- 17: weitere Mikroorganismen, die nicht nachzuweisen sind
- 18: nachzuweisende Mikroorganismen mit einer stark ausgeprägten äußeren Hülle
- 19: Konditionierung gemäß herkömmlichem FISH Verfahren
- 20: Waschen gemäß herkömmlichem FISH-Verfahren
- 21: erfindungsgemäßes Verfahren für Mikroorganismen mit einer stark ausgeprägten äußeren Hülle
- 22: nachzuweisende Mikroorganismen mit einer stark ausgeprägten äußeren Hülle nach einer Konditionierung

## Patentansprüche

1. Verfahren (7) zum Nachweis von Mikroorganismen ohne einer stark ausgeprägten äußeren Hülle (1) in einer Probe (9) mittels Einschleusen (10) spezifischer Nukleinsäuresonden (11) in die einzelnen Mikroorganismen (1), die mit dem vorhandenen Nukleinsäurematerial der Mikroorganismen (1) reagieren (12), und anschließendem optischen Detektieren (16) der in den einzelnen Mikroorganismen (1) erzeugten Reaktionsprodukte, wobei zumindest während dem Einschleusen (10) der Nukleinsäuresonden (11) eine Fixierung (13) der in der Probe (9) enthaltenen Mikroorganismen (1) durchgeführt wird, **dadurch gekennzeichnet, dass** der Probe (9) ein Detergens (14) zugegeben wird, bevor der Fixierungsschritt (13) abgeschlossen ist, insbesondere wobei das Einschleusen (10) und die Fixierung (13) zeitlich parallel erfolgen.

2. Verfahren (21) zum Nachweis von Mikroorganismen mit einer stark ausgeprägten äußeren Hülle (18) in einer Probe (9) mittels Einschleusen (10) spezifischer Nukleinsäuresonden (11) in die einzelnen konditionierten Mikroorganismen (22), die mit dem vorhandenen Nukleinsäurematerial der konditionierten Mikroorganismen (22) reagieren (12), und anschließendem optischen Detektieren (16) der in den einzelnen konditionierten Mikroorganismen (22) erzeugten Reaktionsprodukte, wobei zumindest während dem Einschleusen (10) der Nukleinsäuresonden (11) eine Fixierung (13) der in der Probe (9) enthaltenen Mikroorganismen (1) durchgeführt wird, **dadurch gekennzeichnet, dass** der Probe (9) ein Detergens (14) zugegeben wird, bevor der Fixierungsschritt (13) abgeschlossen ist, insbesondere wobei das Einschleusen (10) und die Fixierung (13) zeitlich parallel erfolgen.

3. Verfahren (7, 21) nach Anspruch 1 und nach Anspruch 2, **dadurch gekennzeichnet, dass** das Detergens (14) in einem Hybridisierungspuffer und/oder in einem Vorbereitungspuffer bereitgehalten wird.

4. Verfahren (7, 21) nach einem der vorangehenden Ansprüche, **dadurch gekennzeichnet, dass** das Detergens (14) ein ionisches Detergens, vorzugsweise Cetyltrimethylammoniumbromid, Natriumdesoxycholat oder Natriumdodecylsulfat (SDS), und/oder ein nicht-ionisches Detergens, vorzugsweise Triton-X-100 oder Tween 80, und/oder ein zwitterionisches Detergens, vorzugsweise CHAPS, ist.

5. Verfahren (7, 21) nach einem der vorangehenden Ansprüche, **dadurch gekennzeichnet, dass** das Detergens (14) nicht vor Beginn der Fixierung (13) und/oder vor Beginn der Fixierung (13), beispielsweise mit der Probe (9) oder in dem Vorbereitungspuffer, zugegeben wird.

6. Verfahren (7, 21) nach einem der vorangehenden Ansprüche, **dadurch gekennzeichnet, dass** der Hybridisierungspuffer mindestens eine denaturierende Substanz und/oder eine Puffersubstanz enthält.

7. Verfahren (7, 21) nach einem der vorangehenden Ansprüche, **dadurch gekennzeichnet, dass** die Fixierung (13) durch die denaturierende Substanz erfolgt.

8. Verfahren (7, 21) nach einem der vorangehenden Ansprüche, **dadurch gekennzeichnet, dass** die denaturierende Substanz eine ungiftige Substanz, insbesondere Guanidiniumchlorid und/oder Harnstoff, und/oder Guanidiniumthiocyanat und/oder Formamid enthält.

9. Verfahren (7, 21) nach einem der vorangehenden Ansprüche, **dadurch gekennzeichnet, dass** der Hybridisierungspuffer mindestens ein oder mehrere Salze, vorzugsweise Natriumchlorid und/oder Magnesiumchlorid, und/oder mindestens einen Chelatbildner, vorzugsweise Ethylendiamintetraessigsäure (EDTA), enthält.

10. Verfahren (7, 21) nach einem der vorangehenden Ansprüche, **dadurch gekennzeichnet, dass** das Detektieren (16) einen Schritt des Quantifizierens und/oder einer Einzeldetektion der Mikroorganismen mit hybridisierten Nukleinsäuresonden umfasst.

11. Verfahren (7, 21) nach einem der vorangehenden Ansprüche, **dadurch gekennzeichnet, dass** die Nukleinsäuresonde (11) komplementär zu einer DNA und/oder RNA eines nachzuweisenden Mikroorganismus (1) ist.

12. Verfahren (7, 21) nach einem der vorangehenden Ansprüche, **dadurch gekennzeichnet, dass** die Nukleinsäuresonde (11) ausgewählt ist aus der Gruppe, bestehend aus monoProbes, dopeProbes, tetraProbes, multiProbes, Molecular Beacons und Scorpions Sonden, wobei die Nukleinsäuresonde (11) insbesondere gequenchte Molecular Beacons umfasst.

13. Verfahren (7, 21) nach einem der vorangehenden Ansprüche, **dadurch gekennzeichnet, dass** die Nukleinsäuresonde (11) mit einem optisch detektierbaren Marker (15) verbunden ist, insbesondere wobei der detektierbare Marker (15) ausgewählt ist aus der Gruppe, bestehend aus Fluoreszenzmarkern, Chemolumineszenzmarkern, Affinitätsmarkern und enzymatisch aktiven Gruppen.

14. Verfahren (7, 21) nach einem der vorangehenden Ansprüche, **dadurch gekennzeichnet, dass** das Verfahren (7) mit einem fluidischen Kanalsystem ausgeführt wird, insbesondere mit einem scheibenförmigen Probenträger.

15. Verfahren (7, 21) nach einem der vorangehenden Ansprüche, **dadurch gekennzeichnet, dass** das Verfahren (7, 21) einen Schritt zur zusätzlichen Hintergrundreduktion, vorzugsweise durch Benutzung eins zweiten Farbstoffs gemäß/analog zum Försterresonanzenergietransfer, und/oder das Einbringen von Quenching-Oligos nach des Annealingschritts der FISH-Sonden, und/oder Immobilisierung von freien FISH-Sonden vor Messung, und/oder physikalische, chemische oder biologische Veränderung von Fluorophoren überschüssiger FISH-Sonden, und/oder Einsatz von "freien" Quencher-Molekülen in Konzentrationen größer als 1mM, und/oder Immobilisierung der Zielorganismen auf Strukturen wie Filtern und insbesondere Track Etched Membranes, und/oder Zusatz bestimmte Reagenzien und insbesondere FISH-Sonden in hohem Überschuss (Metering), umfasst.

16. Verfahren (7, 21) nach einem der vorangehenden Ansprüche, **dadurch gekennzeichnet, dass** das Verfahren (7, 21) einen zusätzlichen Schritt zur verbesserten Lebend/Tot-Unterscheidung, vorzugsweise durch Abbau der Ziel-Nukleinsäuren (wie rRNA) (Target Depletion) in toten Mikroorganismen vor dem eigentlichen Verfahren (7), und/oder Einbringen von Nukleinsäuren (z.B. DNA-Oligos) in tote Mikroorganismen um damit Untersuchungs-relevante Bindungsstellen (z.B. in deren rRNA) (Target Blocking) zu besetzen, und/oder Einsatz von Lebend/Tot-Färbungen, umfasst.

17. Fluidisches Kanalsystem, vorzugsweise scheibenförmiger Probenträger, mit Mitteln zum Durchführen des Verfahrens (7) und Verfahren (21) nach einem der Ansprüche 1 bis 13, insbesondere umfassend mindestens eine Kavität mit einer Nukleinsäuresonde (11) und mindestens einem Detergens (14), und/oder mit Mitteln zur optischen Zählung von markierten Mikroorganismen ohne einer stark ausgeprägten äußeren Hülle (1) und/oder von markierten Mikroorganismen mit einer stark ausgeprägten äußeren Hülle (18)..
